(19) Europäisches Patentamt
European Patent Office
Office européen des brevets

(11) Numéro de publication: **0 097 567**
**B1**

(12) **FASCICULE DE BREVET EUROPEEN**

(45) Date de publication du fascicule du brevet: **31.08.88**

(51) Int. Cl.⁴: **A 61 K 7/06**

(21) Numéro de dépôt: **83401162.9**

(22) Date de dépôt: **08.06.83**

# (54) Utilisation d' alkylphénol polyoxyéthyléné comme agents antiséborrhéiques.

(30) Priorité: **16.06.82 FR 8210509**

(43) Date de publication de la demande:
**04.01.84 Bulletin 84/01**

(45) Mention de la délivrance du brevet:
**31.08.88 Bulletin 88/35**

(84) Etats contractants désignés:
**BE CH DE FR GB IT LI LU NL SE**

(56) Documents cités:
**FR-A-2 293 219**
**FR-A-2 301 233**
**GB-A-1 051 461**
**US-A-3 131 152**
**US-A-3 171 786**
**US-A-3 436 167**

**CHEMICAL ABSTRACTS, vol. 93, no. 26, décembre 1980, page 373, no. 245266q, Columbus, Ohio, USA**

**Dictionnaire Français de Médecine, vol. 14, page 619**

(73) Titulaire: **SANOFI, société anonyme**
**40, Avenue George V**
**F-75008 Paris (FR)**

(72) Inventeur: **Girard, Jacques**
**9 rue Clair Soleil**
**F-34430 St. Jean Vedas (FR)**
Inventeur: **Barbier, Alain**
**280 avenue Miradou Le Belvédère**
**F-34980 St Clement la Riviere (FR)**

(74) Mandataire: **Gillard, Marie-Louise et al**
**Cabinet Beau de Loménie 55, Rue d'Amsterdam**
**F-75008 Paris (FR)**

**EP 0 097 567 B1**

Il est rappelé que: Dans un délai de neuf mois à compter de la date de publication de la mention de la délivrance du brevet européen toute personne peut faire opposition au brevet européen délivré, auprès de l'Office européen des brevets. L'opposition doit être formée par écrit et motivée. Elle n'est réputée formée qu'après paiement de la taxe d'opposition. (Art. 99(1) Convention sur le brevet européen).

Courier Press, Leamington Spa, England.

**Description**

La présente invention concerne l'utilisation comme agents anti-séborrhéiques à usage cosmétique de certains alkylphénols polyoxyéthylénés.

Les compositions dont l'utilisation est l'objet de la présente invention contiennent en tant que principe — actif au moins une substance répondant à la formule générale:

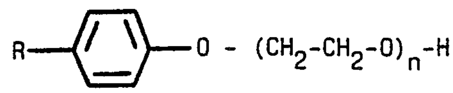 (I)

dans laquelle

R désigne un radical alkyle à chaîne droite ou ramifiée pouvant comporter de 4 à 16 atomes de carbone,

n est un nombre entier pouvant varier de 4 à 40.

Le radical alkyle R linéaire ou ramifé comporte de préférence environ 9 atomes de carbone, c'est pourquoi les dits produits sont souvent désignés sous le nom de nonylphénols polyoxyéthylénes; n est avantageusement compris entre 6 et 12.

Les produits de formule (I) se présentent en général sous forme de mélange dans lesquels plusieurs produits différents sont présents mais ils peuvent également être utilisés à l'état de produits purs.

Ces produits ont été largement utilisés dans des domaines divers essentiellement en fonction de leurs propriétés physiques en tant que détergents, agents mouillants, émulsifiants ou solubilisants.

A cet effet, on peut citer les documents de l'art antérieur ci-après:

— le brevet US 3.131.152 qui décrit des compositions liquides moussantes sous forme d'aérosols, contenant un monoalcool aliphatique inférieur, de l'eau, un surfactant et un agent propulseur. Le surfactant peut être un agent non ionique, anionique ou cationique. Les alkylphénols polyoxyéthylénés sont cités parma les surfactants appropriés. Il est donc clair que le surfactant est utilisé uniquement pour ses propriétés physicochimiques.

— le brevet GB 1.051.461 qui a pour objet des compositions dermatologiques contenant 0,5 à 15% d'une amine $RNH_2$ (où R est une chaine hydrocarbonée droite, saturée ayant 12 à 200 atomes de carbone) dans une base dermatologique acceptable.

Il est précisé que lorsque la base est une lotion ou un shampooing, cette base peut inclure un agent surfactant non ionique.

Il apparaît clairement que la substance active est l'amin $RNH_2$ et que le surfactant présent seulement dans certaines formes, n'intervient que par ses propriétés physicochimiques.

— le brevet US 3.436.167 concerne des compositions pour teinture de cheveux ou shampooing contenant à titre de substance active un surfactant amphotère. Il est précisé que ces compositions peuvent contenir des agents tensio-actifs ou non. La présence optionnelle de ces agents et leur gamme étendue indique qu'ils sont utilisés pour leurs propriétés physicochimiques.

— le brevet US 3.171.786 qui a pour objet des préparations pour le coiffage des cheveux comprenant une solution de polyvinylpyrrolidone et d'un agent opacifiant constitué par un membre de la famille des amides primaires dérivés d'aides gras. Ces dernières substances étant insolubles, il est nécessaire d'ajouter un agent solubilisant qui peut être un nonylphénol polyoxyéthyléné.

Dans ce cas, ce sont les propriétés solubilisants de l'alkylphénol polyoxyéthyléné qui sont mises en jeu.

Le brevet FR 2.293.219 est relatif à un procédé d'inactivation de l'herpès par application locale d'un agent tensio-actif non ionique détruisant la paroi virale. Le nonylphénol polyoxyéthyléné est cité parmi les agents tensio-actifs appropriés. La grande variété des agents tensio-actifs appropriés. La grande variété des agents tensio-actifs utilisables ainsi que les études effectuées laissent penser que l'inactivation est liée plus aux propriétés tensio-actives du produit qu'à sa structure. De plus, on notera que cette inactivation ne présente aucun lien avec l'action sur la séborrhée.

Par ailleurs, on sait que ces alkylphénols polyoxyéthylénés ont été utilisés dans des compositions pharmaceutiques en mélange avec d'autres substances actives.

Par exemple le brevet FR 2.301.233 a pour objet des compositions pharmaceutiques destinées au traitement de l'acné et comprenant cinq constituants différents dans des rapports de poids donnés. Parmi ces constituants, figurent les alkylphénols polyoxyéthylénés qui agissent comme inhibiteurs de lipases.

L'étude de cet art antérieur montre que les alkylphénols polyoxyéthylénés ont été essentiellement utilisés comme agents tensio-actifs ou en mélange avec d'autres substances actives. On n'a donc jamais proposé de les utiliser seuls à titre de principe actif dans des compositions antiséborrhéiques.

Les produits de formule (1), utilisés selon l'invention, peuvent être obtenus par réaction de l'oxyde d'éthylène sur un alkylphénol:

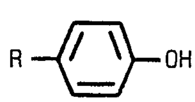

en présence d'un catalyseur approprié. On poursuit la réaction jusqu'à ce que le quantité nécessair (n molécules) d'oxyde d'éthylène ait été absorbée. En pratique, on n'obtient pas un produit unique, mais un mélange de différentes substances dont la proportion moléculaire d'oxyde d'éthylène dans le produit suit la formule de répartition de Poisson. Par suite, dans les produits livrés par les fabricants, l'indication n=X ne signifie pas qu'il s'agit du composé pur où n=X, mais en fait d'un mélange de substances dans lequel la substance n=X est majoritaire, mais qui contient également les substances n=X−1, n=X−2, n=X−3, etc. et n=X+1, n=X+2, n=X+3, etc. en proportions plus faibles.

Les mélanges ainsi obtenus peuvent être utilisés directement, selon l'invention.

Les composés (I) peuvent être obtenus purs à partir d'un phénol pur correspondant, convenablement substitué, sur lequel on fait réagir un dérivé chloré (2):

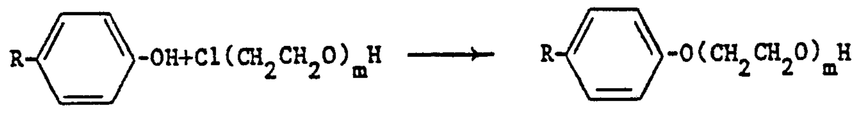

*(1)* *(2)* *(3)*

Les composés (*1*) sont connus en général ou ils peuvent être préparés par les méthodes classiques de préparation des phénols. Les dérivés chlorés (*2*) sont connus jusqu'à m=6 et on peut ainsi obtenir les composés (I) pour lesquels n=4, 5 ou 6.

On prépare tout d'abord un sel alcalin, en général le sel de sodium du phénol à substituer. Celui-ci, repris dans un solvant inerte anhydre tel que le toluène, est chauffé au reflux pendant 15 à 20 heures avec un excès de 25 à 50% du dérivé chloré (*2*).

Après filtration du chlorure de sodium formé, on évapore le solvant puis on élimine les produits volatils (excès de dérivé chloré (*2*)) par chauffage à 250°C sous vide poussé. On purifie le résidu par chromatographie sur gel de silice.

A partir des composes (I) où n=4, 5, 6 ainsi obtenus on peut préparer les composés où n a une valeur plus grande en faisant réagir sur les premiers le dérivé chloré (*2*). La substitution s'effectuant ici sur un hydroxyle alcoolique et non sur un hydroxyle phénolique, il est nécessaire pour éviter des condensations parasites de bloquer l'hydroxyle terminal du dérivé chloré (*2*).

Afin de pouvoir éliminer facilement ensuite ce groupe protecteur, on utilise le plus souvent un groupe tétrahydropyrannyle qui s'élimine par simple chauffage en milieu acide:

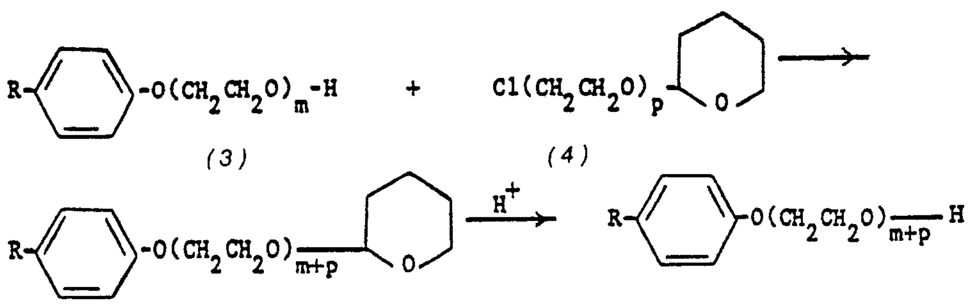

*(3)* *(4)*

*(5)* *(6)*

La condensation est effectuée par chauffage à 60°C des réactifs (*3*) et (*4*) en présence de soude et d'un catalyseur tel que le sulfate acide de tétrabutylammonium.

Après la fin de réaction, le groupe tétrahydropyrannyle est éliminé par chauffage en milieu acide, puis le produit (*6*) est purifié par chromatographie sur silice.

En faisant varier le valeurs de m et p, il est possible d'obtenir tous les produits (I) possédant une valeur de n comprise entre 6 et 12. En répétant la même opération à partir des produits (I) ainsi obtenus on peut préparer les composés (I) où n varie de 12 à 18 et ainsi de suite.

Les réactifs (*4*) s'obtiennent aisément à partir des dérivés chlorés (*2*) par chauffage avec le dihydro-3,4 *2H* pyranne en présence d'acide paratoluène sulfonique.

Les composés (I) "purs" se présentent le plus souvent sous forme d'une huile. Ils sont caractérisés d'une part par leur indice de réfraction et d'autre part par leur temps de rétention en chromatographie HPLC.

Il a donc été trouvé que les prouduits de formule (I) purs ou sous forme de mélanges peuvent être utilisés dans les compositions cosmétiques de lutte contre la séborrhée.

La séborrhée est due à un flux sébacé excessif causé par une hyperactivité de la glande sébacée. L'abondance du sébum qui en résulte, à la surface de la peau, présente des effets indésirables pouvant entraîner des complications telles que l'acné, mais également des eczémas ou des alopécies au niveau du cuir chevelu.

De nombreuses substances ont été présentées pour le traitement de la séborrhée. Toutefois, dans la plupart des cas, elles se sont révélées peu actives par voie topique, ou bien ont donné lieu à des effets secondaires indésirables.

Les compositions utilisés selon l'invention sont efficaces contre la séborrhée sans présenter pour autant d'effets secondaires indésirables. Les produits de formule générale (I) ont fait l'objet d'une étude d'activité antiséborrhéique ainsi que d'une étude complémentaire de leurs autres propriétés tant par voie générale qu'appliqués directement sur la peau.

## I—ACTIVITE ANTISEBORRHEIQUE

### A) Activité sur la lipogénèse sébacée in vitro

Méthode:

Après sacrifice au chloroforme de rats mâles Sprague Dawley, d'un poids moyen de 210 g, les oreilles sont découpées aux ciseaux suivant des repères anatomiques fixes, puis leur côté ventral est soigneusement rasé. Après fixation à l'aide de rubans adhésifs sur une plaque de verre, le côté ventral de l'oreille est prélévé à l'aide d'un kératotome de Castroviejo dont l'épaisseur de coupe est réglée sur 3 mm. Ce prélèvement est ensuite coupé en petits carrés de 1 $mm^2$ avec une lame de rasoir et pesé avant introduction dans le tube servant à l'incubation.

L'incubation se fait dans 1,5 ml de Krebs-Ringer tamponné à pH 7,4 (tampon phosphate Sörensen 1/15 M) et contenant 100 U/ml de pénicilline, 1 mg/ml de Streptomycine et 2 mmol de glucose non radioactif. Le produit, à la concentration à étudier, est préalablement dissous dans du propylèneglycol (concentration finale égale à 0,5%), puis introduit dans le milieu d'incubation. Les tubes servant à l'incubation sont maintenus dans un bain de glace, jusqu'à ce que tous les prélèvements soient effectués, puis 7,4kBq de glucose U—$^{14}C$ (74—148 MBq/mmol) sont ajoutés dans chaque tube.

Pour chaque rat utilisé, une des oreilles sert à tester le produit antiséborrhéique potentiel, alors que l'autre sert de témoin.

Après 4 heures d'incubation à 37°C, le milieu d'incubation est éliminé, puis l'incubat subit 4 lavages de 3 mins avec agitation à l'aide de glucose non radioactif (2 g/l). Une fois la dernière eau de lavage éliminée, 3 ml de potasse alcoolique N sont rajoutés dans chaque tube contenant les glandes sébacées de la peau d'oreille. Les tubes sont bouchés et portés à 70°C pendant une nuit.

Après ce temps, les tubes sont refroidis et 3 ml d'acide chlorhydrique 2N sont introduits dans chaque tube. Les lipides présents correspondant aux glandes sébacées de chaque oreille sont alors extraits par 2 fois 4 ml d'hexane après agitation de 5 min. Après séparation des phases aqueuse et hexanique, 3,25 ml d'hexane à la première extraction et 4 ml à la deuxième sont prélevés et réunis dans une fiole de comptage contenant 10 ml de liquide de mélange scintillant.

Les fioles, après bouchage et agitation, sont introduites dans un compteur à scintillation liquide préréglé sur le canal $^{14}C$. Le temps de comptage de chaque fiole est de 4 min. Pour chaque fiole, le résultat exprimé en coups/minute (cpm) est transformé en fonction du poids de prélèvement en cpm/mg et l'inhibition (ou l'augmentation) de la synthèse des lipides est donnée par la variation % (Δ%) de l'incorporation en cpm/mg entre l'oreille témoin et l'oreille "traitée".

La moyenne des Δ% est effectuée et la valeur statistique des résultats est déterminée par le calcul du coefficient "t" de Student.

### B) Activité antiséborrhéique par voie locale

Méthode:

Des hamsters femelles Syrien doré, d'un poids moyen de 110 g, sont tondus et rasés au niveau des organes du flanc qui se présentent sous l'aspect d'une tache sombre de 2 mm de diamètre environ, de part et d'autre de la colonne vertébrale. Sur un des organes, 1,25 µg de testostérone propionate est appliqué journellement sous un volume de 10 µl d'acétone, l'autre organe recevent la même quantité de testostérone associée au produit à étudier. Le traitement s'effectue 5 jours/7 jours pendant 21 jours.

A la fin de cette période, les animaux sont sacrifiés au chloroforme et les diamètres des taches pigmentées sont measurés à l'aide d'une régle graduée. La peau est prélevée, fixée côte épiderme vers le bas, sur une planche en bois. Le muscle peaucier et le tissu adipeux sont alors éliminés par rabotage au Kératotome de Castroviejo (épaisseur de coupe réglée à 3 mm), puis une biopsie de 10 mm de diamètre au niveau de chaque organe est réalisée à l'aide d'un trépan. Cette biopsie est débitée en carrés de 1 $mm^2$, puis pesée (±0,1 mg).

Le reste de manipulation: incubation, saponification, extraction, est identique à celui effectué dans le test de lipogénèse in vitro ci-dessus.

## II—EFFET ANTI-INFLAMMATOIRE

### Inflammation à l'huile de croton chez le rat

La méthode utilisée est celle de Tonelli et Coll., telle qu'elle est décrite par Le Douarec et Visalli (J. Pharmacol., Paris, 1979, *1* (3), 395—405).

## III—TOXICITE ET TOLERANCE

### 1) Toxicité aiguë

Bien que destinée à une utilisation topique, la toxicité aiguë des différents produits a été déterminée par voie orale et par voie sous-cutanée.

Protocole expérimental:

La toxicité aiguë des produits (I) est recherchée chez la souris après traitement par voies sous-cutanée et orale.

Des souris femelles COBS CD1, d'un poids moyen de 20 g, sont réparties au hasard en lots de 6 animaux. Les produits sont mis en solution dans l'eau distillée à différentes concentrations puis administrés par les deux voies sous un volume de 0,1 à 0,4 ml/10 g de poids corporel.

Les souris sont observées le jour même et pendant les 7 jours suivants. La mortalité est notée dans les différents lots.

### 2) Tolérance locale et toxicité transcutanée chez le cobaye

Methode:

L'étude a été réalisée sur des groupes de 6 cobayes mâles Hartley, d'un poids de 500 g environ.

Quatre zones de 4 $cm^2$ sont préparées à la tondeuse électrique à peigne fin, de part et d'autre de la ligne médiane dorsale, et entretenues régulièrement tous les 2 jours. Le produit est appliqué une fois par jour par massage d'une durée de 90 secondes avec un excès de préparation, le côte gauche recevant le produit à la concentration à étudier (5, 10 ou 20%), le côte droit le placebo correspondant (alcool à 95°).

Le traitement a été appliqué durant 3 semaines à raison de 6 jours/7 jours. Les observations sur le plan cutané ont porté sur la présence ou non d'érythème ou d'hyperkératose.

On a, en outre, contrôlé l'influence éventuelle du traitement sur la croissance pondérale des animaux.

## IV—EFFETS HORMONAUX

Les test hormonaux utilisés sont des tests classiques: activité oestrogène et anti-oestrogène activité androgène et anabolisante, activité anti-androgène, activité progestative et activité antigonadotrope.

Les produits ont été administrés par voie sous-cutanée à la dose unique de 100 mg/kg et par jour chez les animaux impubères.

Dans les différents tests effectués, les produits (I) présentent une activité hormonale nulle ou très faible qui, en aucun cas, n'est susceptible de s'opposer à leur utilisation par voie topique.

Les exemples et résultats non limitatifs ci-après illustrent l'invention.

### Exemple 1

Préparation d'un produit (I) pur dans lequel R est $(CH_3)—(CH_2)_8—$ et n est égal à 6. (CM 40124)

A la solution de 10 g de nonyl-4 phénol dans du méthanol on ajoute par petites fractions 1,3 g de sodium puis on chauffe 1 heure au reflux.

On évapore le méthanol à siccité et on reprend le sel de sodium dans 150 ml de toluène anhydre. On ajoute 18 g de chloro-17 pentaoxa-3,6,9,12,15 heptadécanol-1 et on chauffe au reflux pendant 20 heures. On filtre le précipité de chlorure de sodium et distille les produits volatils par chauffage à 220°C sous vide poussé.

On purifie le produit par chromatographie sur colonne de silice en éluant par un mélange chloroforme-méthanol.

On obtient un liquide visqueux hygroscopique présentant un indice de réfraction $n_D^{22}=1,4891$ et un temps de rétention HPLC de 7 min.

### Exemple 2

Préparation d'un produit (I) pur dans lequel R est $(CH_3)—(CH_2)_8$ et n est égal à 12. (CM 7886)

a) *(chloro-17 pentaoxa-3,6,9,12,15 heptadécyloxy)-2 tétrahydropyranne*

On chauffe sous agitation à 50°C pendant 1 heure, le mélange de 133 g de chloro-17 pentaoxa-3,6,9,12,15 heptadécanol-1 45 g de dihydro-3,4 *2H* pyranne et 0,2 g d'acide paratoluène sulfonique.

Après refroidissement, on reprend le mélange réactionnel dans 200 ml d'éther et neutralise par addition d'une solution de soude à 10%. On filtre l'insoluble et évapore le solvant à siccité.

On obtient 170 g du produit attendu caractérisé par l'absence de bande OH dans le spectre infrarouge.

b) *CM 7886*

On forme une émulsion par agitation d'un mélange de:

25 g de nonyl-4 phénoxy-17 pentaoxa-3,6,9,12,15 heptadécanol-1 (produit de l'exemple 1),

25 g de chloro-17 pentaoxa-3,6,9,12,15 heptadécyloxy-2 tétrahydropyranne,

20 g d'une solution aqueuse de soude à 50%,

1,1 g de bisulfate de tétrabutylammonium,

puis on chauffe à 80°C pendant 16 heures.

Après refroidissement on reprend le résidu dans 200 ml d'eau et on extrait 3 fois avec du chloroforme. On lave la solution organique avec de l'eau, on sèche sur sulfate de sodium et on évapore le solvant à siccité.

On reprend le résidu avec 300 ml de méthanol, on ajoute 1 ml d'acide chlorhydrique concentré et on chauffe à 60°C pendant 1 heure. On évapore le solvant puis on élimine les produits volatils en chauffant à 240°C sous 6,67 Pa (0,05 mm de mercure).

On chromatographie le résidu sur une colonne de silice (15 g de silice par gramme de produit) en éluant avec du chloroforme puis avec un mélange de chloroforme et de méthanol. (97%—3%). On récupère ainsi 8 g du produit dans lequel R est $(CH_3)-(CH_2)_8-$ et n est égal à 12.

Ce produit est un solide (point de fusion: 30°C).

On ne décrit pas la méthode connue en elle-même de préparation de mélange de produits de formule (I).

Les résultants des tests concernant les propriétés des produits sont rapportés ci-après.

Activité sur la lipogénèse sébacée in vitro

a) Les résultats obtenus sur les produits "purs" sont consignés dans le tableau I ci-après.

b) Les résultats obtenus sur des "mélanges" sont consignés dans le tableau II ci-après. Ces résutats ont tous été obtenus à l'aide de produits pour lesquels R est en moyenne en $C_9$ (nonyl, etc.).

Activité antiséborrhéique locale

a) Avec le produit 7886 et avec une dose de 2000 µg par jour on a obtenu par rapport au témoin, une inhibition de 100% de la lipogenèse sébacée.

b) Avec les mélanges de produits de formula I dans lesquels R est en moyenne en $C_9$, on a obtenu les résultats présentés dans le tableau III ci-après.

Activité anti-inflammatoire—inflammation à l'huile de croton

a) Avec les produits purs on a obtenu les résultats présentés dans le tableau IV ci-après.

b) Avec les mélanges de produits de formule I dans lequels R est en moyenne en $C_9$ on a obtenu les résultats présentés dans le tableau V ci-après.

Tolérance locale et toxicité

a) Les produits purs de formule I présentent une faible toxicité et une bonne tolérance locale.

b) Les mélanges de produits de formule I dans lesquels R est en moyenne en $C_9$ présentant:

des résultats de toxicité rapportés dans le tableau VI ci-après,

des résultats de tolérance locale rapportés dans le tableau VII ci-après.

En résumé, les différents résultats obtenus montrent que les produits (I):

possèdent une bonne activité antiséborrhéique associée en général à une activité anti-inflammatoire topique modérée;

sont dénués d'effets, secondaires gênants, en particulier d'effets hormonaux;

présentent une faible toxicité par voie générale et une bonne tolérance locale jusqu'à concentration de 10 à 20%.

Les composés (I) présentent donc toutes les propriétés requises pour la réalisation de compositions cosmétiques anti-séborrhéiques.

Ces compositions peuvent être présentées sous toute forme convenant à l'application externe, telle que, par exemple, lotion, crème, gel shampooing, etc.

On indiquera ci-après, à titre non limitatif, certaines compositions cosmétiques réalisables avec les divers produits de formule (I)

A) Avec utilisation de "produits purs" (de formule I)

*solution hydroalcoolique*

| | | |
|---|---|---|
| CM 7887 ($R=C_9H_{19}-n=12$) | | 10 g |
| alcool éthylique à 60° | qsp | 100 g |

*shampooing*

| | | |
|---|---|---|
| CM 7887 ($R=C_9H_{19}-n=12$) | | 15 g |
| dihydroxyméthyllauramide | | 5 g |
| essence de lavande | | 0,2 g |
| soude | qsp | pH 7,5 |
| eau purifiée | qsp | 100 g |

B) Avec utilisation de mélanges de produits dans lesquels R est en moyenne en $C_9$

*solution hydroalcoolique*

| | | |
|---|---|---|
| CM 7617 (n=12) | | 10 g |
| alcool éthylique à 60° | qsp | 100 g |

*gel aqueux*

| | | |
|---|---|---|
| CM 7646 (n=14) | | 5 g |
| Carbopol 934 | | 1 g |
| soude | qsp | formation du gel |
| eau purifiée | qsp | 100 g |

*gel alcoolique*

| | | |
|---|---|---|
| CM 7617 (n=12) | | 3 g |
| Carbopol 934 | | 1,5 g |
| propylèneglycol | | 10 g |
| triéthanolamine | qsp | pH 7 |
| alcool éthylique à 70° | qsp | 100 g |

*gel alcoolique*

| | | |
|---|---|---|
| CM 7646 (n=14) | | 5 g |
| Carbopol 934 | | 1,5 g |
| triéthylamine | qsp | formation du gel |
| alcool éthylique à 70° | qsp | 100 g |

*shampooing*

| | | |
|---|---|---|
| CM 7723 (n=9) | | 10 à 20 g |
| dihydroxyméthyllauramide | | 3 à 7 g |
| essence de lavande | | 0,2 g |
| soude | qsp | pH 7,5 |
| eau purifiée | qsp | 100 g |

*shampooing*

| | | |
|---|---|---|
| CM 7722 (n=6) | | 15 à 20 g |
| tétracimate disodique | | 0,100 g |
| essence de lavande | | 0,200 g |
| acide lactique | qsp | pH 5,2 |
| eau purifiée | qsp | 100 g |

TABLEAU I

ACTIVITE SUR LA LIPOGENESE "IN VITRO"

| N°de Code | R | n | Concentration % | Synthèse des lipides à partir de glucose $(U\text{-}^{14}C)\Delta$ % témoin |
|---|---|---|---|---|
| 40 181 | 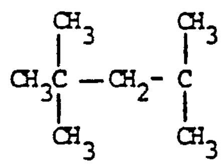 | 6 | 0,01<br>0,005<br>0,0025<br>0,001 | - 67 *<br>- 42 *<br>- 5<br>- 4 |
| 40 182 | " | 12 | 0,005<br>0,0025<br>0,001<br>0,0005 | - 89 *<br>- 69 *<br>- 41 *<br>- 19 |
| 40 124 | $CH_3(CH_2)_8$ | 6 | 0,01<br>0,005<br>0,0025<br>0,001 | - 55 *<br>- 42 *<br>- 31 *<br>- 6 |
| 7948 | $CH_3(CH_2)_8$— | 9 | 0,01<br>0,005<br>0,0025<br>0,001 | - 81 *<br>- 61 *<br>- 31 *<br>- 10 |
| 7886 | $CH_3(CH_2)_8$— | 12 | 0,005<br>0,025<br>0,001<br>0,0005<br>0,00025 | - 84 *<br>- 71 *<br>- 51 *<br>- 33 *<br>- 6 |

**T A B L E A U  I I**

**Activité sur la lipogénèse sébacée in vitro.**

| N° de code du produit | n (moles d'oxyde d'éthylène) | Concentration % | Synthèse des lipides à partir de glucose (U - $^{14}C$) Δ%/témoin |
|---|---|---|---|
| CM 7616 | 4 | 0,005 | -20 * |
| CM 7721 | 5 | 0,01<br>0,005<br>0,0025<br>0,001 | -65 *<br>-49 *<br>-26 *<br>- 2 |
| CM 7722 | 6 | 0,01<br>0,005<br>0,0025<br>0,001 | -80 *<br>-57 *<br>-40 *<br>-12 |
| CM 7723 | 9 | 0,005<br>0,0025<br>0,001<br>0,0005 | -78 *<br>-47 *<br>-19<br>-11 |
| CM 7750 | 10 | 0,01<br>0,005<br>0,0025<br>0,001 | -88 *<br>-73 *<br>-46 *<br>-16 |
| CM 7617 | 12 | 0,005<br>0,0025<br>0,001<br>0,0005<br>0,00025 | -73 *<br>-45 *<br>-32 *<br>-31 *<br>+ 8 |
| CM 7646 | 14 | 0,005<br>0,0025<br>0,001<br>0,0005<br>0,00025 | -78 *<br>-35 *<br>-31 *<br>-16<br>+ 5 |
| CM 7751 | 20 | 0,01<br>0,005<br>0,0025<br>0,001 | -66 *<br>-50 *<br>-28 *<br>- 6 |

* p ⩽ 0,005 (t de Student)

## TABLEAU III

Activité antiséborrhéique locale.

| N° de code du produit | n (moles d'oxyde d'éthylène) | Dose (µg/j/21 j) | Lipogénèse à partir de glucose (U- $^{14}C$) Δ%/témoin |
|---|---|---|---|
| CM 7616 | 4 | 2 000 | -42 * |
| CM 7721 | 5 | 2 000 | -18 |
| CM 7722 | 6 | 2 000 | -62 * |
| CM 7723 | 9 | 2 000 | -67 * |
| CM 7750 | 10 | 2 000 | -32 |
| CM 7617 | 12 | 2 000<br>1 000<br>500<br>250 | -51 *<br>-62 *<br>-45 *<br>+26 |
| CM 7646 | 14 | 2 000 | -61 * |
| CM 7751 | 20 | 2 000 | -26 |

* p ≤ 0,05 (t de Student)

## TABLEAU IV

INFLAMMATION A L'HUILE DE CROTON CHEZ LE RAT

| N° de Code | R | n | Concentration % | % de variation par rapport aux témoins |
|---|---|---|---|---|
| 7886 | $CH_3-(CH_2)_8-$ | 12 | 10<br>5<br>2,5<br>1,25<br>0,625<br>0,312 | - 81 *<br>- 49 *<br>- 43 *<br>- 15 *<br>- 19 *<br>+ 3 |
| 7887 | $C_9H_{19}$ | 12 | 5<br>2,5<br>1,25<br>0,625 | - 55 *<br>- 31 *<br>- 22 *<br>0 |
| 40257 | $C_9H_{19}$ | 18 | 5<br>2,5<br>1,25<br>0,625<br>0,312 | - 80 *<br>- 48 *<br>- 22 *<br>- 32 *<br>- 11 |

TABLEAU V

Inflammation à l'huile de croton chez le rat.

| n° de code du produit | Concentration, en % | % de variation par rapport aux témoins |
|---|---|---|
| CM 7616 (n = 4) | 10<br>5<br>2,5<br>1,25<br>0,625 | -56 *<br>-38 *<br>-25 *<br>-11<br>- 4 |
| CM 7721 (n = 5) | 10<br>5<br>2,5<br>1,25 | -49 *<br>-30 *<br>-22 *<br>- 6 |
| CM 7722 (n = 6) | 10<br>5<br>2,5<br>1,25<br>0,625 | -57 *<br>-37 *<br>-18<br>-14<br>+ 2 |
| CM 7723 (n = 9) | 10<br>5<br>2,5<br>1,25<br>0,625 | -83 *<br>-37 *<br>-18<br>- 8<br>- 1 |
| CM 7750 (n = 10) | 10<br>5<br>2,5<br>1,25<br>0,625 | -91 *<br>-79 *<br>-52 *<br>-29 *<br>-16 |

TABLEAU V (suite)

| n° de code du produit | Concentration, en % | % de variation par rapport aux témoins |
|---|---|---|
| CM 7617 (n = 12) | 10<br>5<br>2,5<br>1,25<br>0,625 | -80 *<br>-57 *<br>-40 *<br>-15<br>- 1 |
| CM 7646 (n = 14) | 10<br>5<br>2,5<br>1,25 | -82 *<br>-62 *<br>-37 *<br>+11 |
| CM 7751 (n = 20) | 10<br>5<br>2,5<br>1,25<br>0,625 | -86 *<br>-76 *<br>-49 *<br>-37 *<br>-10 |
| CM 7752 (n = 30) | 10<br>5<br>2,5<br>1,25<br>0,625<br>0,312 | -81 *<br>-66 *<br>-60 *<br>-55 *<br>-30 *<br>-14 |
| CM 7765 (n = 40) | 10<br>5<br>2,5<br>1,25<br>0,625 | -73 *<br>-52 *<br>-40 *<br>-16<br>+ 1 |

* $p \leqslant 0{,}05$ (W de Wilcoxon)

TABLEAU VI

Toxicité aiguë chez la souris.

| n° de code du produit | Voie sous-cutané<br>Doses létales (mg/kg) | | | Voie orale<br>Doses létales (mg/kg) | | |
|---|---|---|---|---|---|---|
| | $DL_0$ | $DL_{50}$ | $DL_{100}$ | $DL_0$ | $DL_{50}$ | $DL_{100}$ |
| CM 7616 (n = 4) | > 1 250 | - | - | 1 250 | 5 000 | > 5 000 |
| CM 7721 (n = 5) | 1 250 | entre 1 250 et 2 500 | > 2 500 | 1 875 | vers 2 500 | > 3 750 |
| CM 7722 (n = 6) | 1 000 | 1 250 | > 2 500 | 1 250 | 2 500 | ≥ 3 750 |
| CM 7723 (n = 9) | 625 | entre 1 000 et 1 250 | ≥ 1 250 | 1 250 | entre 2 500 et 3 750 | ≤ 3 750 |
| CM 7750 (n = 10) | 625 | vers 950 | 1 250 | 1 250 | vers 2 500 | 3 750 |
| CM 7617 (n = 12) | 800 | entre 800 et 1 000 | 1 000 | 2 500 | entre 2 500 et 5 000 | > 5 000 |
| CM 7646 (n = 14) | 250 | entre 625 et 1 250 | > 1 250 | 2 500 | entre 2 500 et 5 000 | 5 000 |
| CM 7751 (n = 20) | 250 | entre 625 et 1 250 | ≤ 1 250 | 1 250 | entre 3 750 et 5 000 | ≥ 5 000 |
| CM 7752 (n = 30) | 1 250 | vers 2 500 | > 2 500 | ≥ 5 000 | - | - |

-$DL_0$ : dose maximale tolérée pour laquelle on n'observe pas de mortalité.

-$DL_{50}$ : dose qui tue 50% des animaux d'un lot.

-$DL_{100}$ : dose qui tue 100% des animaux d'un lot.

TABLEAU VII

Tolérance locale chez le cobaye.

(3 semaines)

| Produit/ alcool 95° | Concentration, % | Tolérance cutanée du produit | Gain pondéral en % jour 17/jour 0 | |
|---|---|---|---|---|
| | | | produit | témoin |
| CM 7723 (n = 9) | 5 | bien toléré | +12 | +17 |
| | 10 | bien toléré | +14 | +17 |
| | 20 | bien toléré | +14 | +17 |
| CM 7617 (n = 12) | 5 | bien toléré | +12 | +18 |
| | 10 | bien toléré | +30 | +31 |
| | 20 | léger érythème bien toléré | +42 | +46 |
| CM 7646 (n = 14) | 5 | bien toléré | +11 | +18 |
| | 10 | bien toléré | +29 | +31 |
| | 20 | mal toléré dès le 4e jour | Arrêt traitement le 8e jour | |

**Revendications**

1. Utilisation comme agent antiséborrhéique, d'une substance ou d'un mélange de substances, répondant à la formule générale:

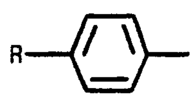 O - $(CH_2-CH_2-O)_n$-H (I)

dans laquelle:

R désigne un radical alkyle à chaîne droite ou ramifiée pouvant comporter de 4 à 16 atomes de carbone,

n est un nombre entier pouvant varier de 4 à 40.

2. Utilisation selon la revendication 1, de substance(s) répondant à la formule générale (I) dans laquelle le radical R comporte en moyenne 9 atomes de carbone.

3. Utilisation selon l'une quelconque des revendications 1 ou 2 pour la préparation de compositions contenant environ 2 g à environ 25 g de substance(s) de formule (I) pour 100 g de composition.

4. Utilisation d'une substance ou d'un mélange de substances répondant à la formule générale:

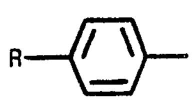 O - $(CH_2-CH_2-O)_n$-H (I)

dans laquelle:

R désigne un radical alkyle à chaîne droite ou ramifiée pouvant comporte de 4 à 16 atomes de carbone,

n est un nombre entier pouvant varier de 4 à 40, pour la préparation de compositions antiséborrhéiques.

5. Utilisation selon la revendication 4 de substance(s) répondant à la formule générale (I) dans laquelle le radical R comporte en moyenne 9 atomes de carbone.

**Patentansprüche**

1. Verwendung einer Substanz oder eines Substanzengemisches der allgemeinen Formel (I) als Anti-Schuppenmittel

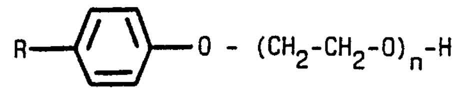

(I)

in der bedeuten:

R einen geradkettigen oder verzweigten Alkylrest mit 4 bis 16 Kohlenstoffatom und

n eine ganze Zahl von 4 bis 40.

2. Verwendung nach Anspruch 1 der Substanz(en) der allgemeinen Formel (I), in der R im Mittel 9 Kohlenstoffatome aufweist.

3. Verwendung nach Anspruch 1 oder 2 zur Herstellung von Zusammensetzungen mit etwa 2 bis 25 g Substanz(en) der Formel (I) je 100 g Zusammensetzung.

4. Verwendung einer Substanz oder eines Substanzengemisches der allgemeinen Formel (I)

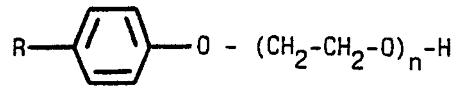

(I)

in der bedeuten:

R einen geradkettigen oder verzweigten Alkylrest mit 4 bis 16 Kohlenstoffatomen und

n eine ganze Zahl von 4 bis 40,

zur Herstellung von Anti-Schuppen-Zusammensetzungen.

5. Verwendung nach Anspruch 4 der Substanz(en) der allgemeinen Formel (I), in der der Rest R im Mittel 9 Kohlenstoffatome aufweist.

**Claims**

1. Use, as an antiseborrheic agent, of a substance or a mixture of substances corresponding to the general formula:

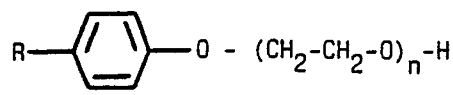

(I)

in which

R denotes a straigh-chain or branched-chain alkyl radical which can contain from 4 to 16 carbon atoms and

n is an integer which can vary from 4 to 40.

2. Use according to claim 1, of a substance or substances corresponding to the general formula (I), in which the radical R contains an average 9 carbon atoms.

3. Use according to either of claims 1 or 2 for the preparation of compositions containing about 2 g to about 25 g of substance or substances of the formula (I) per 100 g of composition.

4. Use of a substance or of a mixture of substances corresponding to the general formula:

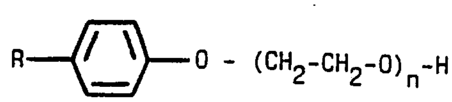

(I)

in which

R denotes a straight-chain or branched-chain alkyl radical which can contain from 4 to 16 carbon atoms and

n is an integer which can vary from 4 to 40, for the preparation of antiseborrheic compositions.

5. Use according to claim 4 of a substance or substances corresponding to the general formula (I) in which the radical R contains an average 9 carbon atoms.